(19) Europäisches Patentamt
European Patent Office
Office européen des brevets

(11) Numéro de publication: 0 267 828 B1

# (12) FASCICULE DE BREVET EUROPEEN

(45) Date de publication du fascicule du brevet: **27.12.90**

(51) Int. Cl.5: **C07C 53/21**, C07C 233/12

(21) Numéro de dépôt: **87402304.7**

(22) Date de dépôt: **15.10.87**

(54) **Composés polyfluorés et leur procédé de préparation.**

(30) Priorité: **24.10.86 FR 8614811**
**17.02.87 FR 8702009**

(43) Date de publication de la demande:
**18.05.88 Bulletin 88/20**

(45) Mention de la délivrance du brevet:
**27.12.90 Bulletin 90/52**

(84) Etats contractants désignés:
**AT BE CH DE FR GB GR IT LI LU NL SE**

(56) Documents cités:
**DE-A- 3 219 075**
**FR-A- 2 103 459**
**FR-A- 2 197 865**
**US-A- 2 701 814**
**US-A- 2 951 051**

(73) Titulaire: **ATOCHEM, 4 & 8, Cours Michelet La Défense 10, F-92800 Puteaux(FR)**

(72) Inventeur: **Gautier, Martine, 14 Allée Paul Sabatier Bâtiment B, F-31000 Toulouse(FR)**
Inventeur: **Rico, Isabelle, 12, rue Garcia Lorca, F-31520 Ramonville(FR)**
Inventeur: **Lattes, Armand, 12, rue Garcia Lorca, F-31520 Ramonville(FR)**
Inventeur: **Bertocchio, René, 49 bis, rue des Vallières, Vourles F-69390 Vernaison(FR)**

(74) Mandataire: **Leboulenger, Jean et al, ATOCHEM Département Propriété Industrielle, F-92091 Paris la Défense 10 Cédex 42(FR)**

EP 0 267 828 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux composés contenant à la fois une chaîne perfluorée ($R_F$) et une chaîne hydrogénée ($R_H$), utilisables notamment comme agents tensioactifs ou comme intermédiaires pour la synthèse de tels agents.

Grâce à leur propriété de diminuer considérablement la tension superficielle des solutions aqueuses, les tensioactifs fluorés ont trouvé une application particulièrement importante dans l'élaboration de mélanges extincteurs destinés à la lutte contre les incendies et notamment ceux d'hydrocarbures. Dans les compositions extinctrices, les tensioactifs fluorés sont généralement associés à des tensioactifs non fluorés de façon à former rapidement, sur la surface de l'hydrocarbure non miscible à l'eau, un film résistant et durable. Bien que des résultats intéressants soient obtenus avec des mélanges de tensioactifs fluorés et non fluorés, il existe un besoin constant de simplifier la formulation des compositions extinctrices, par exemple en proposant un tensioactif unique contenant simultanément une chaîne perfluorée et une chaîne hydrogénée.

Dans cette optique, la présente invention a maintenant pour objet des composés mixtes, contenant une chaîne perfluorée et une chaîne hydrogénée.

Les composés selon l'invention peuvent être représentés par la formule générale :

$$R_F-(CH_2)_m-\underset{\displaystyle CO-X}{\underset{|}{CH}}-R_H \qquad (I)$$

dans laquelle $R_F$ représente un radical perfluoroalkyle, linéaire ou ramifié, contenant de 1 à 20 atomes de carbone, $R_H$ représente un radical alkyle, linéaire ou ramifié, contenant de 4 à 18 atomes de carbone, m est un nombre entier allant de 0 à 2, et X représente un groupement OH ou $NR_1R_2$ dans lequel $R_1$ et $R_2$ peuvent être identiques ou différents et représentent chacun un atome d'hydrogène ou un radical méthyle.

Sont particulièrement préférés les composés dont les radicaux $R_F$ et $R_H$ contiennent de 4 à 12 atomes de carbone et dont l'azote n'est pas substitué ($R_1$=$R_2$=H).

L'invention inclut aussi les mélanges de deux composés de ce type, l'un répondant à la formule :

$$R_F-(CH_2)_{m'}-\underset{\displaystyle CO-X}{\underset{|}{CH}}-C_nH_{2n+1} \qquad (I\text{-}a)$$

et l'autre à la formule :

$$R_F-(CH_2)_{m'-1}-\underset{\displaystyle CO-X}{\underset{|}{CH}}-C_{n+1}H_{2n+3} \qquad (I\text{-}b)$$

où m' est égal à 1 ou 2 et n est un nombre entier allant de 4 à 17, le rapport molaire I-a/I-b pouvant aller de 0,1 à 10.

Les composés selon l'invention dans lesquels X est un groupement $NR_1R_2$ peuvent être préparés par photoamidation d'une oléfine polyfluorée de formule générale :

$R_F$-$(CH_2)_p$-CH=CH-$C_nH_{2n+1}$ (II)

dans laquelle p est égal à 0 ou 1 et n est un nombre entier allant de 4 à 17, en présence d'un excès de formamide ou d'un dérivé méthylé de celui-ci.

Cette réaction est de préférence réalisée par irradiation aux ultraviolets d'un mélange contenant l'oléfine polyfluorée (II) et le formamide ou son dérivé méthylé en présence d'acétone comme initiateur. L'opération peut être effectuée en milieu homogène constitué par une solution des réactifs (oléfine, formamide et acétone) dans un solvant organique inerte tel que le méthyl-2 propanol-1, le dioxanne-1,4 et, de préférence, le tert- butanol.

Sauf dans le cas du diméthylformamide, on peut également soumettre aux rayonnements UV une microémulsion des réactifs obtenue à l'aide d'un agent tensioactif non ionique et d'un agent cotensioactif du type alcool, l'un de ces deux agents devant être fluoré et l'autre non fluoré. Comme agents tensioactifs non ioniques utilisables, on peut citer plus particulièrement les alkylphénols éthoxylés, les polycondensats d'oxyde d'éthylène et de propylène (Pluronics) et les polyfluoroalcools éthoxylés. Comme exemples de contensio actifs, on peut citer les alcools aliphatiques tels que l'hexanol, l'heptanol et l'octanol, et les alcools fluoroaliphatiques du type $R_FC_2H_4OH$ tels que le perfluorobutyl-2 éthanol et ses homologues perfluorohexyl et perfluorooctyl. Pour obtenir une microémulsion convenable, il est indispensable d'utiliser le tensioactif non ionique et le cotensioactif en quantités telles que leur rapport massique soit compris entre 0,5 et 1,5 et de préférence sensiblement égal à 1. A cet égard, conviennent particulièrement bien l'association d'un condensat du nonylphénol avec 5 à 14 moles d'oxyde d'éthylène et du perfluorobutyl-2 éthanol, ainsi que l'association d'un condensat du perfluorohexyl-2 éthanol avec 10 à 15 moles d'oxyde d'éthylène et de l'hexanol-1, de l'heptanol-1 ou de l'octanol-1.

La durée de l'irradiation, de préférence effectuée à une température allant de 25 à 50°C, peut varier dans de larges limites en fonction de l'intensité de l'irradiation et est généralement comprise entre 12 et 60 heures. On obtient les meilleurs rendements pour des durées d'irradiation allant de 30 à 50 heures.

Comme indiqué précédemment, on opère en présence d'un excès de formamide ou de son dérivé méthylé. Le rapport molaire formamide (ou dérivé méthylé)/oléfine (II) peut varier dans de larges limites, mais pour favoriser la réaction de photoamidation et améliorer le rendement, il est généralement compris entre 10 et 120, de préférence entre 40 et 90.

La quantité d'acétone à utiliser comme initiateur peut elle aussi varier dans de larges limites. De préférence, on choisira cette quantité de telle sorte que le rapport molaire acétone/formamide soit compris entre 0,03 et 0,2 et avantageusement entre 0.05 et 0.09.

Quelque soit la méthode choisie (milieu homogène ou microémulsion), la photoamidation d'une oléfine de formule II conduit à un mélange des deux amides de formules :

$$R_F-(CH_2)_{m'}-\underset{\displaystyle CONR_1R_2}{\underset{|}{CH}}-C_nH_{2n+1} \qquad (III\text{-}a)$$

$$R_F-(CH_2)_{m'-1}-\underset{\displaystyle CONR_1R_2}{\underset{|}{CH}}-C_{n+1}H_{2n+3} \qquad (III\text{-}b)$$

les symboles m', n, $R_F$, $R_1$ et $R_2$ ayant les mêmes significations que précédemment.

Toutefois, lorsqu'on opère en milieu homogène, on obtient toujours majoritairement l'amide III-a correspondant à la fixation du groupement carbamoyle sur le carbone oléfinique le plus éloigné de la chaîne perfluorée. En microémulsion , le produit majoritaire est le plus souvent l'amide III-b correspondant à l'amidation du carbone oléfinique le plus proche de la chaîne perfluorée. Si on le désire, les amides III-a et III-b peuvent être séparés par les méthodes usuelles telles que l'extraction par des solvants fluorés (par exemple le trichlorotrifluoroéthane) ou la chromatographie préparative.

Les oléfines polyfluorées (II) de départ sont des produits connus. Celles dans lesquelles p est égal à zéro, c'est-à-dire les oléfines $R_F-CH=CH-C_nH_{2n+1}$, peuvent généralement être obtenues par addition d'un iodure de perfluoroalkyle $R_FI$ sur une oléfine $C_nH_{2n+1}CH=CH_2$ (voir G.V.D TIERS, J. Org. Chem. 27, 2261, 1962 et N.O. BRACE, J. Org. Chem. 37, 2429, 1972), puis déhydroiodation par la potasse alcoolique de la iodhydrine formée $R_FCH_2CHI-C_nH_{2n+1}$. Celles dans lesquelles p est égal à 1, c'est-à-dire les oléfines $R_F-CH_2-CH=CH-C_nH_{2n+1}$, peuvent être préparées par réaction d'un aldéhyde aliphatique sur un iodure de perfluoroalkyl-2 éthyl triphénylphosphonium, lui-même obtenu en faisant réagir la triphénylphosphine sur un iodure de perfluoroalkyl-2 éthyle (voir B. ESCOULA et al,. Synth. Comm. 15(1),35,1985 et C. CECUTTI et al, J. Dispersion Science and Technology, 7(3), 307, 1986).

Les composés selon l'invention dans lesquels X est un groupement OH peuvent être préparés par hydrolyse des amides. Cette hydrolyse peut être effectuée en milieu aqueux au moyen d'une base qui est de préférence l'hydroxyde de sodium mais peut être également l'hydroxyde de potassium. On opère en milieu basique fort 12 à 36 N, de préférence 20 à 30 N à une température pouvant aller de 100 à 160°C, mais de préférence comprise entre 120 et 140°C. On considère que l'hydrolyse est terminée au bout de 24 heures. Aprés acidification du mélange réactionnel, l'acide formé peut être isolé par les méthodes habituelles, notamment par extraction à l'aide d'un solvant choisi parmi les hydrocarbures chlorofluorés tels que, par exemple, le trichloro-trifluoro-éthane.

Les acides selon l'invention se présentent sous forme d'huiles ou de solides. Ils peuvent être facilement transformés en sels solubles dans l'eau par les méthodes classiques, par exemple pour les sels de potassium par action d'une solution de potasse éthanolique.

Les acides polyfluorés selon l'invention et leurs sels peuvent être utilisés comme agents tensioactifs ou, ainsi que les amides, comme intermédiaires de synthèse de tels agents.

Les exemples suivants illustrent l'invention sans la limiter. La référence utilisée pour les spectres RMN $^{19}F$ est l'acide trifluoroacétique.

<u>EXEMPLE 1</u> : Préparation des nonafluoro-1,1,1,2,2,3,3,4,4 pentadécane-carboxamides-6 et 7

Dans un tube en pyrex on introduit 0,744 g de l'oléfine fluorée $C_4F_9$-$CH_2$-CH=CH-$C_8H_{17}$, 5,88 ml de formamide, 15,84 ml de tert-butanol et 0,83 ml d'acétone. Le mélange est ensuite dégazé par un courant d'argon pendant 5 minutes pour éliminer l'oxygène de l'air, puis le tube est placé sur un manège tournant dans une enceinte circulaire recouverte de 16 lampes UV (λ=300 nm ; P=16 W) qui maintiennent une température d'environ 30-35°C. On laisse le tube aux ultraviolets pendant deux jours.

On élimine ensuite par filtration le précipité d'oxamide formé au cours de la synthèse, puis on évapore le filtrat sous vide (2000 Pa) et reprend le résidu avec 5 ml d'eau. Il se forme un précipité qu'on purifie par recristallisation dans un mélange de volumes égaux d'acétone et d'éther de pétrole, puis sèche sur $P_2O_5$ dans un dessicateur (666 Pa).

On obtient ainsi 0,57 g d'un solide qui fond à 80°C et dont les analyses (RMN $^1H$, RMN $^{19}F$ et CPV) révèlant qu'il correspond aux isomères de formules :

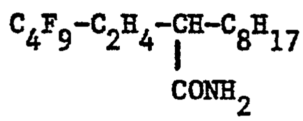 et 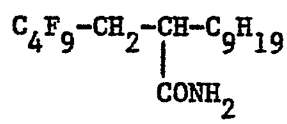

(a) (b)

dans le rapport a/b égal à 2,89.

<u>EXEMPLE 2</u> : Préparation des tridécafluoro-1,1,1,2,2,3,3,4,4,5,5,6,6 tridécane-carboxamides-8 et 9

On opère comme à l'exemple 1, mais avec 0,832 g de l'oléfine fluorée $C_6F_{13}$-$CH_2$-CH=CH-$C_4H_9$. On obtient 0,58 g d'un solide qui fond à 78°C et dont les analyses RMN et CPV montrent qu'il correspond aux isomères de formules :

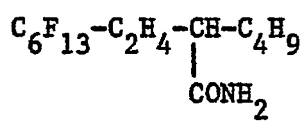 et 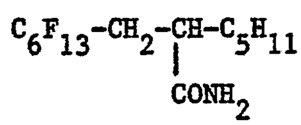

(a) (b)

dans le rapport a/b égal à 1,39.

<u>EXEMPLE 3</u> : Préparation des heptadécafluoro-1,1,1,2,2,3,3,4,4,5,5, 6,6,7,7,8,8 pentadécane-carboxamides-10 et 11

En opérant comme à l'exemple 1 à partir de 1,032 g de l'oléfine fluorée $C_8F_{17}$-$CH_2$-CH=CH-$C_4H_9$, on obtient 0,98 g d'un solide qui fond à 88°C et dont les analyses RMN et CPV révélent qu'il correspond aux isomères de formules :

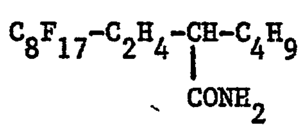 et 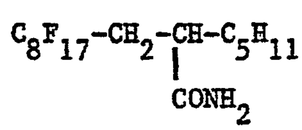

(a) (b)

dans le rapport a/b égal à 1,22.

<u>EXEMPLE 4</u> : Préparation des heptadécafluoro-1,1,1,2,2,3,3,4,4,5,5, 6,6,7,7,8,8 eicosane-carboxamides-9 et 10

En opérant comme à l'exemple 1 à partir de 1,2 g de l'oléfine fluorée $C_8F_{17}$-CH=CH-$C_{10}H_{21}$, on obtient 0,82 g d'un solide qui fond à 92°C et correspond aux isomères de formules :

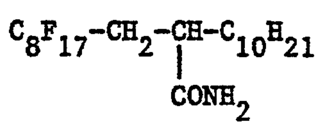 et 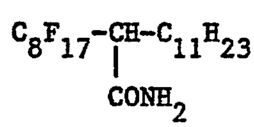

(a) (b)

dans le rapport a/b égal à 7,71.

EXEMPLE 5 : Préparation des nonafluoro-1,1,1,2,2,3,3,4,4 pentadécane-carboxamides-6 et 7

On introduit dans un tube en pyrex une microémulsion préparée à partir de :

- 0,26 g de l'oléfine fluorée $C_4F_9$-$CH_2$-CH=CH -$C_8H_{17}$
- 1,48 g de formamide
- 1,63 g de perfluorobutyl-2 éthanol
- 1,63 g de nonylphénol polythoxylé (10,5 moles d'oxyde d'éthylène par mole de nonylphénol)
- 0,18 ml d'acétone

Aprés dégazage à l'argon et irradiation pendant deux jours comme à l'exemple 1, l'injection en CPV de la microémulsion montre la présence des isomères suivants :

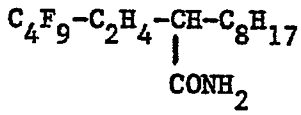
et
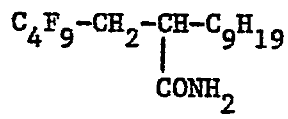

(a) (b)

dans un rapport a/b égal à 0,95.

EXEMPLE 6 : Préparation des heptadécafluoro-1,1,1,2,2,3,3,4,4,5,5, 6,6,7,7,8,8 eicosane-carboxamides-9 et 10

Pour préparer les amides :

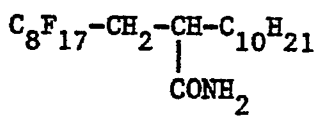
et
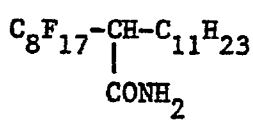

(a) (b)

on opère comme à l'exemple 5 avec 0,26 g de l'oléfine fluorée $C_8F_{17}$-CH=CH-$C_{10}H_{21}$ en utilisant 1,63 g de quatre nonylphénols polyéthoxylés différant entre eux par le nombre (EON) de motifs oxyde d'éthylène.

Les résultats obtenus sont rassemblés dans le tableau suivant :

| E O N | Rapport a/b | Rendement (%) |
|---|---|---|
| 6 | 0,42 | 71,3 |
| 8 | 0,74 | 31,5 |
| 10 | 0,40 | 80,4 |
| 12 | 0,44 | 88,4 |

Dans une autre série d'essais avec la même oléfine fluorée $C_8F_{17}$-CH=CH-$C_{10}H_{21}$, on a remplacé, d'une part, le nonylphénol polyéthoxylé par la même quantité (1,63 g) du tensioactif fluoré non ionique $C_6F_{13}C_2H_4(OC_2H_4)_{12}$-OH et, d'autre part, le perfluorobutyl-2 éthanol par la même quantité (1,63 g) de différents alcools hydrogénés (hexanol-1, heptanol-1 et octanol-1). Les résultats obtenus figurent dans le tableau suivant :

| Alcool hydrogéné | Rapport $\frac{a}{b}$ | Rendement (%) |
|---|---|---|
| Hexanol-1 | 0,25 | 87,8 |
| Heptanol-1 | 0,26 | 87,7 |
| Octanol-1 | 0,29 | 84,2 |

EXEMPLE 7 : Préparation des heptadécafluoro-1,1,1,2,2,3,3,4,4,5,5, 6,6,7,7,8,8 eicosane-N-méthyl-carboxamides-9 et 10

En opérant comme à l'exemple 1 à partir de 1,2 g de l'oléfine fluorée $C_8F_{17}$-CH=CH-$C_{10}H_{21}$, et 8,65 ml de N-méthyl-formamide, on obtient 1,1 g d'une pâte qui fond à une température inférieure à 50°C qui correspond aux isomères :

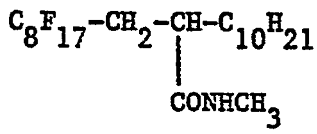 et 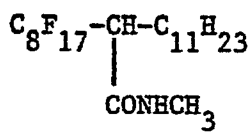

(a) (b)

dans un rapport a/b égal à 1,08.

EXEMPLE 8 : Préparation des heptadécafluoro-1,1,2,2,3,3,4,4,5,5, 6,6,7,7,8,8 eicosane-N-méthyl-carboxamides-9 et 10

On introduit dans un tube en pyrex une microémulsion préparée à partir de :

- 0,26 g de l'oléfine fluorée $C_8F_{17}$-CH=CH-$C_{10}H_{21}$
- 1,48 g de N-méthyl-formamide
- 1,63 g de perfluorobutyl-2 éthanol
- 1,63 g de nonylphénol polythoxylé (12 moles d'oxyde d'éthylène par mole de nonylphénol)
- 0,18 ml d'acétone

Aprés dégazage à l'argon et irradiation pendant deux jours comme à l'exemple 1, l'injection de la microémulsion montre la présence des isomères suivants :

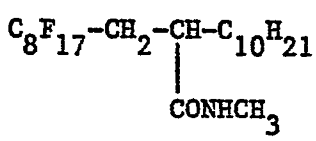 et 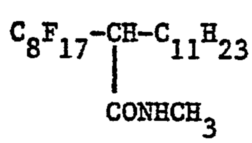

(a) (b)

dans un rapport a/b égal à 0,55.

EXEMPLE 9 : Préparation des heptadécafluoro-1,1,1,2,2,2,3,3,4,4,5,5, 6,6,7,7,8,8 eicosane-N,N-diméthyl-carboxamides-9 et 10

En opérant comme à l'exemple 1 à partir de 1,2 g d'oléfine fluorée $C_8F_{17}$-CH=CH-$C_{10}H_{21}$ et 11,4 ml de N,N-diméthyl-formamide, on obtient 1,11 g d'une huile qui correspond aux isomères de formules :

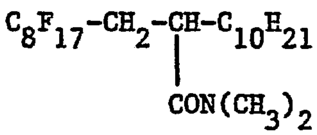 et 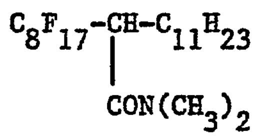

(a) (b)

dans un rapport a/b égal à 1,01.

L'oléfine $C_8F_{17}$-CH=CH-$C_{10}H_{21}$ utilisée dans les exemples 4 et 6-9 a été préparée en opérant comme suit : 546 g d'iodure de perfluorooctyle et 5 g d'azobisisobutyronitrile sont placés dans un réacteur de 1 litre équipé d'un agitateur, d'un réfrigérant à reflux et d'une ampoule de coulée. On porte le mélange à 75°C, puis on introduit en une heure 261 g de dodécène-1. Aprés 6 heures de réaction, on ajoute 2,5 g d'azobisisobutyronitrile, puis on poursuit le chauffage à 75°C pendant 4 heures. On distille ensuite le mélange réactionnel pour éliminer l'excès d'iodure de perfluorooctyle et de dodécène (110-160°C sous 2666 Pa), puis aux 678 g de produit restant en ajoute en 4 heures une solution de 68 g d'hydroxyde de potassium dans 250 ml d'éthanol, tout en maintenant la temp érature à 40-45°C. Aprés addition de 1 litre d'eau et décantation, on sépare la phase organique (563 g) et purifie l'oléfine ainsi obtenue par distillation sous vide (120°C/133 Pa).

EXEMPLE 10 : Préparation des acides nonafluoro-1,1,1,2,2,3,3,4,4 pentadécane-carboxyliques-6 et 7

Dans un ballon surmonté d'un réfrigérant à reflux, on chauffe à 130°C pendant 24 heures un mélange de 125 mg du solide obtenu à l'exemple 1, 2 ml d'eau et 2 g d'hydroxyde de sodium en pastilles. Aprés refroidissement, le pH du mélange réactionnel est ramené à 1 par addition de 4,2 ml d'acide chlorhydrique à 37 % et laissé sous agitation pendant 3 heures.

Par extraction de la phase aqueuse à l'aide du trichloro-1,1,2 trifluoro-1,2,2 éthane, puis évaporation du solvant, on obtient 52 mg d'une huile jaune dont les analyses montrent qu'il s'agit des acides de formules :

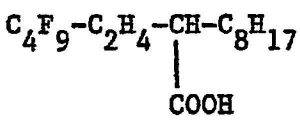 et 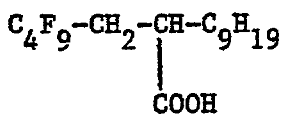

Spectre IR : 2960 (OH libre) ; 1711 (C=O)
1460 (C-H α $CO_2H$) ; 1236 (C-F)

Spectre RMN $^1H$ ($CD_3COCD_3$) : 0,77 (3 p, m, $CH_3$)
1,16 (14 p, m, $CH_2$)
2,14 (1 p, m, CH)
3,25 (4 p, m, $CH_2$ α et β $CF_2$)

Spectre RMN $^{19}F$ ($CD_3COCD_3$): -5,59 (3 f, m, $CF_3$)
-38,83 (2 f, m, $CF_2$ α $CH_2$)
- 48,61 (2 f, m, $CF_2$ β $CH_2$)
- 50,36 (2 f, m, $CF_2$ α $CF_3$)

EXEMPLE 11 : Préparation des acides tridécafluoro-1,1,1,2,2,3,3,4, 4,5,5,6,6 tridécane-carboxyliques-8 et 9

On opère comme à l'exemple 10, mais avec 138 mg du solide obtenu à l'exemple 2. On récupère 100 mg d'une huile jaune constituée par les acides :

$$C_6F_{13}-C_2H_4-\underset{\displaystyle COOH}{\underset{|}{CH}}-C_4H_9 \quad \text{et} \quad C_6F_{13}-CH_2-\underset{\displaystyle COOH}{\underset{|}{CH}}-C_5H_{11}$$

Spectre IR : 2969 (OH libre) ; 1714 (C=O)

1460 (CH α $CO_2H$) ; 1208 (C-F)

EXEMPLE 12 : Préparation des acides heptadécafluoro-1,1,1,2,2,3,3, 4,4,5,5,6,6,7,7,8,8 eicosane-carboxylique-9 et 10

En opérant comme à l'exemple 10 avec 189 mg du solide de l'exemple 4, on obtient 63 mg d'une huile jaune constituée par les acides:

$$C_8F_{17}-CH_2-\underset{\displaystyle COOH}{\underset{|}{CH}}-C_{10}H_{21} \quad \text{et} \quad C_8F_{17}-\underset{\displaystyle COOH}{\underset{|}{CH}}-C_{11}H_{23}$$

Spectre IR : 2960 (OH libre) ; 1720 (C=O)

1470 (CH α $CO_2H$) ; 1200 (C-F)

Spectre RMN $^1H$ ($CD_3COCD_3$) : 0,92 (3 p, m, $CH_3$)

1,30 (18 p, m, $CH_2$)

2 (1 p, m, CH)

2,87 (2 p, m, $CH_2$ α $CF_2$)

Spectre RMN $^{19}F$ ($CD_3COCD_3$) : - 5,27 (3 f, m, $CF_3$)

- 37,34 (2 f, m, $CF_2$ α $CH_2$)

- 46,06 (6 f, m, $CF_2$)

- 46,69 (2 f, m, $CF_2$ γ $CF_3$)

- 47,75 (2 f, m, $CF_2$ β $CF_3$)

- 50,35 (2 f, m, $CF_2$ α $CF_3$)

**Revendications**

1. Composés polyfluorés, caractérisés en ce qu'ils répondent à la formule générale:

$$R_F-(CH_2)_m-\underset{\displaystyle CO-X}{\underset{|}{CH}}-R_H \qquad (I)$$

dans laquelle $R_F$ représente un radical perfluoroalkyle, linéaire ou ramifié, contenant de 1 à 20 atomes de carbone, $R_H$ représente un radical alkyle, linéaire ou ramifié, contenant de 4 à 18 atomes de carbone, m est un nombre entier allant de 0 à 2, et X représente un groupement OH ou $NR_1R_2$ dans lequel $R_1$ et $R_2$, identiques ou différents, représentent chacun un atome d'hydrogène ou un radical méthyle.

2. Composés selon la revendication 1, dans lesquels $R_1$ et $R_2$ sont des atomes d'hydrogéne.

3. Composés selon la revendication 1 ou 2, dans lesquels les radicaux $R_F$ et $R_H$ contiennent chacun de 4 à 12 atomes de carbone.

4. Mélanges de deux composés selon l'une des revendications 1 à 3, caractérisés en ce que l'un des

composés répond a la formule :

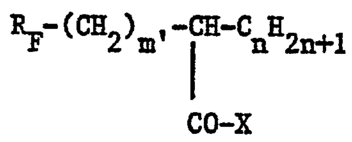

(I-a)

et l'autre à la formule:

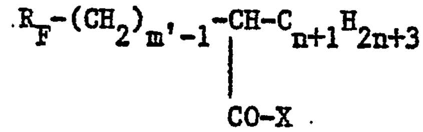

(I-b)

formules dans lesquelles m' est égal à 1 ou 2 et n est un nombre entier allant de 4 à 17, le rapport molaire I-a/I-b pouvant aller de 0,1 à 10.

5. Procédé de préparation des composés polyfluorés selon la revendication 1, caractérisé en ce que l'on soumet une oléfine polyfluorée de formule générale:

$R_F-(CH_2)_p-CH=CH-C_nH_{2n+1}$

dans laquelle p est égal à 0 ou 1 et n est un nombre entier allant de 4 à 17, à une photoamidation en présence d'un excès de formamide ou d'un dérivé méthylé de celui-ci et, éventuellement, on soumet l'amide obtenue à une hydrolyse.

6. Procédé selon la revendication 5, dans lequel on soumet à une irradiation aux ultraviolets un mélange contenant l'oléfine (II) et le formamide ou son dérivé méthylé, en présence d'acétone comme initiateur.

7. Procédé selon la revendication 6, dans lequel on opère en milieu homogène constitué par une solution de l'oléfine (II), du formamide et d'acétone dans un solvant organique inerte.

8. Procédé selon la revendication 7, dans lequel le solvant est le tert- butanol.

9. Procédé selon la revendication 6, dans lequel on soumet à l'action d'un rayonnement W une microémulsion de l'oléfine (II), du formamide et d'acétone, obtenue à l'aide d'un agent tensioactif non ionique et d'un agent cotensioactif du type alcool, l'un de ces deux agents étant fluoré et l'autre non fluoré, le tensioactif non ionique et le cotensioactif étant utilisés en quantités telles que leur rapport massique soit compris entre 0,5 et 1,5.

10. Procédé selon la revendication 9, dans lequel l'agent tensioactif non ionique est choisi parmi les alkylphénols éthoxylés, les polycondensats d'oxyde d'éthylène et de propyléne, et les polyfluoroalcools éthoxylés.

11. Procédé selon la revendication 9 ou 10, dans lequel l'agent cotensioactif est un alcool aliphatique ou fluoroaliphatique.

12. Procédé selon la revendication 9, dans lequel l'agent tensioactif non ionique est un condensat du nonylphénol avec 5 à 14 moles d'oxyde d'éthylène et l'agent cotensioactif est le perfluorobutyl-2 éthanol.

13. Procédé selon la revendication 9, dans lequel l'agent tensioactif non ionique est un condensat du perfluorohexyl-2 éthanol avec 10 à 15 moles d'oxyde d'éthylène et l'agent cotensioactif est l'hexanol-1, l'heptanol-1 ou l'octanol-1.

14. Procédé selon l'une des revendications 5 à 13, dans lequel le rapport molaire formamide/oléfine (II) est compris entre 10 et 120, de préférence entre 40 et 90.

15. Procédé selon l'une des revendications 6 à 14, dans lequel le rapport molaire acétone/formamide est compris entre 0,03 et 0,2 de préférence entre 0.05 et 0,09.

## Patentansprüche

1. Polyfluorierte Verbindungen, dadurch gekennzeichnet, daß sie der allgemeinen Formel

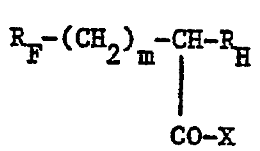

(I)

entsprechen, worin $R_F$ einen geradkettigen oder verzweigten, 1 bis 20 Kohlenstoffatome enthaltenden Perfluoralkylrest bedeutet, $R_H$ einen geradkettigen oder verzweigten, 4 bis 18 Kohlenstoffatome enthaltenden Alkylrest bedeutet, m eine ganze Zahl von 0 bis 2 ist und X eine OH- oder $NR_1R_2$-Gruppe bedeutet, in der $R_1$ und $R_2$ identisch oder verschieden sind und jeweils ein Wasserstoffatom oder einen Methylrest bedeuten.

2. Verbindungen nach Anspruch 1, dadurch gekennzeichnet, daß $R_1$ und $R_2$ Wasserstoffatome sind.

3. Verbindungen nach Anspruch 1 oder 2, dadurch gekennzeichnet, daß die Reste $R_F$ und $R_H$ jeweils 4 bis 12 Kohlenstoffatome enthalten.

4. Gemische von zwei Verbindungen nach einem der Ansprüche 1 bis 3, dadurch gekennzeichnet, daß eine der Verbindungen der Formel

$$R_F-(CH_2)_{m'}-\underset{\displaystyle CO-X}{\underset{|}{CH}}-C_nH_{2n+1} \qquad (I\text{-}a)$$

und die andere der Formel

$$R_F-(CH_2)_{m'-1}-\underset{\displaystyle CO-X}{\underset{|}{CH}}-C_{n+1}H_{2n+3} \qquad (I\text{-}b)$$

entspricht, in denen m' gleich 1 oder 2 und n eine ganze Zahl von 4 bis 17 ist und das Molverhältnis von I-a/I-b 0, 1 bis 10 betragen kann.

5. Verfahren zur Herstellung der polyfluorierten Verbindungen nach Anspruch 1, dadurch gekennzeichnet, daß man ein polyfluoriertes Olefin der allgemeinen Formel

$R_F-(CH_2)_p-CH=CH-C_nH_{2n+1}$

worin p gleich 0 oder 1 und n eine ganze Zahl von 4 bis 17 ist, einer Photoamidierung in Gegenwart eines Überschusses an Formamid oder eines seiner Methylderivate unterzieht und gegebenenfalls das erhaltene Amid hydrolysiert.

6. Verfahren nach Anspruch 5, dadurch gekennzeichnet, daß man ein Gemisch, das das Olefin (II) und Formamid oder sein Methylderivat enthält, in Gegenwart von Aceton als Initiator einer Ultraviolett-Bestrahlung unterzieht.

7. Verfahren nach Anspruch 6, dadurch gekennzeichnet, daß man in einem homogenen Milieu arbeitet, das aus einer Lösung des Olefins (II), des Formamids und des Acetons in einem inerten organischen Losungsmittel besteht.

8. Verfahren nach Anspruch 7, dadurch gekennzeichnet, daß das Lösungsmittel tert.-Butanol ist.

9. Verfahren nach Anspruch 6, dadurch gekennzeichnet, daß man eine UV-Strahlung auf eine Mikroemulsion des Olefins (II), des Formamids und des Acetons einwirken läßt, die mit Hilfe eines nicht-ionischen oberflächenaktiven Mittels und eines Cotensids vom Alkohol-Typ erhalten wurde, wobei eines dieser zwei Mittel fluoriert ist, und das nicht-ionische oberflächenaktive Mittel und das Cotensid in solchen Mengen eingesetzt werden, daß ihr Masseverhältnis zwischen 0,5 und 1,5 beträgt.

10. Verfahren nach Anspruch 9, dadurch gekennzeichnet, daß das nicht-ionische oberflächenaktive Mittel aus den ethoxylierten Alkylphenolen, den Ethylen und Propylenoxidpolykondensaten und den ethoxylierten Polyfluoralkoholen ausgewählt ist.

11. Verfahren nach Anspruch 9 oder 10, dadurch gekennzeichnet, daß das Cotensid ein aliphatischer oder fluoraliphatischer Alkohol ist.

12. Verfahren nach Anspruch 9, dadurch gekennzeichnet, daß das nicht-ionische oberflächenaktive Mittel ein Kondensat des Nonylphenols mit 5 bis 14 mol Ethylenoxid und das Cotensid 2-Perfluorbutylethanol ist.

13. Verfahren nach Anspruch 9, dadurch gekennzeichnet, daß das nicht-ionische oberflächenaktive Mittel ein Kondensat des 2-Perfluorhexylethanols mit 10 bis 15 mol Ethylenoxid und das Cotensid 1-Hexanol, 1-Heptanol oder 1-Octanol ist.

14. Verfahren nach einem der Ansprüche 5 bis 13, dadurch gekennzeichnet, daß das Molverhältnis Formamid/Olefin (II) zwischen 10 und 120, vorzugsweise zwischen 40 und 90 ist.

15. Verfahren nach einem der Ansprüche 6 bis 14, dadurch gekennzeichnet, daß das Molverhältnis Aceton/Formamid zwischen 0,03 und 0,2, vorzugsweise zwischen 0,05 und 0,09 ist.

**Claims**

1. Polyfluoro compounds characterized in that they correspond to the general formula:

$$R_F-(CH_2)_m-\underset{\displaystyle CO-X}{\underset{|}{CH}}-R_H \qquad (I)$$

in which $R_F$ denotes a linear or branched perfluoroalkyl radical containing from 1 to 20 carbon atoms, $R_H$ denotes a linear or branched alkyl radical containing from 4 to 18 carbon atoms, m is an integer ranging from 0 to 2 and X denotes an OH or $NR_1R_2$ group in which each of $R_1$ and $R_2$, which are identical or different, denotes a hydrogen atom or a methyl radical.

2. Compounds according to Claim 1, in which $R_1$ and $R_2$ are hydrogen atoms.

3. Compounds according to Claim 1 or 2, in which each of the radicals $R_F$ and $R_H$ contains from 4 to 12 carbon atoms.

4. Mixtures of two compounds according to one of Claims 1 to 3, characterized in that one of the compounds corresponds to the formula:

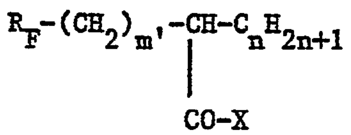

(I-a)

and the other to the formula:

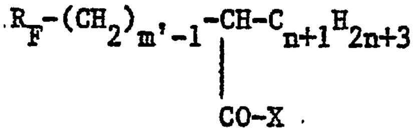

(I-b)

in which formulae m' is equal to 1 or 2 and n is an integer ranging from 4 to 17, it being possible for the molar ratio I-a/I-b to range from 0.1 to 10.

5. Process for the preparation of the polyfluoro compounds according to Claim 1, characterized in that a polyfluoro olefin of general formula:

$R_F-(CH_2)_p-CH=CH-C_nH_{2n+1}$ (II)

in which p is equal to 0 or 1 and n is an integer ranging from 4 to 17, is subjected to a photoamidation in the presence of an excess of formamide or of a methylated derivative thereof and, optionally, the amide obtained i8 subjected to a hydrolysis.

6. Process according to Claim 5, in which a mixture containing the olefin (II) and the formamide or its methylated derivative is subjected to an irradiation with ultraviolet rays in the presence of acetone as initiator. 7. Process according to Claim 6, in which the operation is carried out in a homogeneous medium consisting of a solution of the olefin (II), of the formamide and of acetone in an inert organic solvent.

8. Process according to Claim 7, in which the solvent is tert-butanol.

9. Process according to Claim 6, in which a microemulsion of the olefin (II), of formamide and of acetone, obtained with the aid of a nonionic surface-active agent and of a cosurface-active agent of the alcohol type is subjected to the action of an W radiation, one of these two agents being fluorinated and the other not fluorinated, the nonionic surfactant and the cosurfactant being employed in such quantities that their mass ratio ig between 0.5 and 1.5.

10. Process according to Claim 9, in which the nonionic surface-active agent is chosen from ethoxylated alkylphenols, polycondensates of propylene and ethylene oxide and ethoxylated polyfluoro alcohols.

11. Process according to Claim 9 or 10, in which the cosurface-active agent is an aliphatic or fluoroaliphatic alcohol.

12. Process according to Claim 9, in which the nonionic surface-active agent is a condensate of nonylphenol with 5 to 14 moles of ethylene oxide and the cosurface-active agent is 2-perfluorobutylethanol.

13. Process according to Claim 9, in which the nonionic surface-active agent is a condensate of 2-perfluorohexylethanol with 10 to 15 moles of ethylene oxide and the cosurface-active agent is 1-hexanol, 1-heptanol or 1-octanol.

14. Process according to one of Claims 5 to 13, in which the molar ratio formamide/olefin (II) is between 10 and 120, preferably between 40 and 90.

15. Process according to one of Claims 6 to 14, in which the molar ratio acetone/formamide is between 0.03 and 0.2, preferably between 0.05 and 0.09.